(19) Europäisches Patentamt
European Patent Office
Office européen des brevets

(11) Veröffentlichungsnummer : **0 357 164 B1**

(12) **EUROPÄISCHE PATENTSCHRIFT**

(45) Veröffentlichungstag der Patentschrift :
23.10.91 Patentblatt 91/43

(51) Int. Cl.⁵ : **G01S 7/52, A61B 8/00**

(21) Anmeldenummer : 89250017.4

(22) Anmeldetag : 23.08.89

(54) Ultraschallverfahren und Schaltungen zu deren Durchführung.

(30) Priorität : 01.09.88 DE 3829999

(43) Veröffentlichungstag der Anmeldung :
07.03.90 Patentblatt 90/10

(45) Bekanntmachung des Hinweises auf die
Patenterteilung :
23.10.91 Patentblatt 91/43

(84) Benannte Vertragsstaaten :
AT BE CH DE ES FR GB GR IT LI LU NL SE

(56) Entgegenhaltungen :
EP-A- 0 064 399
EP-A- 0 072 330
EP-A- 0 147 955
DE-A- 3 702 355
US-A- 3 640 271
JOURNAL OF THE ACOUSTICAL SOCIETY OF
AMERICA. vol. 69, no. 4, April 1981, NEW
YORK US Seiten 1210 - 1212; W.K.Law et al.:
"Ultrasonic determination of the nonlinearity
parameter B/A for biological media" &
"METHODS"

(56) Entgegenhaltungen :
JOURNAL OF THE ACOUSTICAL SOCIETY OF
AMERICA. vol. 83, no. 3, März 1988, NEW
YORK US Seiten 942 - 949; L.Germain et al.:
"Generation and detection of high-order harmonics in liquids using a scanning acoustic
microscope"

(73) Patentinhaber : SCHERING
AKTIENGESELLSCHAFT Berlin und
Bergkamen
Müllerstrasse 170/178 Postfach 65 03 11
W-1000 Berlin 65 (DE)

(72) Erfinder : Uhlendorf, Volkmar, Dr.
Eichenallee 30
W-1000 Berlin 19 (DE)
Erfinder : Fritzsch,Thomas,Dr.
Elisenstrasse 2
W-1000 Berlin 41 (DE)
Erfinder : Siegert, Joachim, Dr.
Weddingstrasse 5
W-1000 Berlin 65 (DE)

(74) Vertreter : Fuchs, Jürgen H., Dr.-Ing. et al
Dr. Fuchs, Dr. Luderschmidt, Dipl.-Phys. Seids,
Dr. Mehler Patentanwälte
Abraham-Lincoln-Strasse 7 Postfach 46 60
W-6200 Wiesbaden (DE)

## Beschreibung

Die Erfindung betrifft Ultraschallverfahren zur Bilddarstellung und gegebenenfalls zur Auswertung eines Dopplerspektrums von begrenzt gegen Schallintensität resistenten Objekten und Schaltungen zu deren Durchführung.

In der Ultraschalltechnik werden Ultraschallwellen in ein Untersuchungsgebiet zur selektiven Bilddarstellung und/oder Auswertung des Dopplerspektrums eingestrahlt. Bei den Verfahren und Geräten zur Werkstoffprüfung und zur Untersuchung biologischer Gewebe werden üblicherweise kombinierte Sende-/Empfangsschallköpfe verwendet. Durch die Kristalle der Schwinger und die Geräteelektronik wird dabei eine Schallfrequenz ($f_o$) festgelegt, die für das Senden und Empfangen gleich ist. Ein typischer 5 MHz-Schallkopf hat einen Frequenzbereich von etwa 3 bis 7 MHZ mit einem Maximum bei $f_o$ = 5 MHz. Im gleichen Frequenzbereich wird beim Puls-Echo-Verfahren das reflektierte und/oder zurückgestreute Signal empfangen. Solche Geräte und Verfahren werden auch bei der Untersuchung biologischer Gewebe unter Einsatz von Ultraschallkontrastmitteln angewendet. Außerhalb des vorgegebenen Frequenzbereiches liegende Signalanteile, wie beispielsweise in einem harmonischen Verhältnis zur Sendefrequenz stehende Schwingungen, werden für die bildliche Darstellung des Untersuchungsobjektes und andere Analysen, wie z.B. Dopplermessungen, nicht genutzt. Bei den bisher bekannten Verfahren und Gerätesystemen werden ferner zur Überdeckung eines größeren Frequenzbereiches mehrere Schallköpfe verwendet, die während der Untersuchung gewechselt werden.

Aus der EP-A2-0147955 ist ein Ultraschallverfahren bekannt, bei dem das Untersuchungsobjekt mit einem Meßimpuls hoher Frequenz und einem Pumpimpuls niedriger Frequenz, aber hohem Schalldruck beschallt wird.

Bei der Auswertung wird die Druckabhängigkeit der Schallgeschwindigkeit ausgenutzt. Der Druck im zu untersuchenden Objekt wird durch den Pumpimpuls variiert.

Der Meßimpuls, der sich mit dem Pumpimpuls überlagert, erfährt hierbei eine Phasenänderung, die letztendlich zur Auswertung herangezogen wird. Entscheidend ist bei diesem bekannten Verfahren somit die Phasenlage. Eine Auswertung der harmonischen, subharmonischen oder ultraharmonischen Frequenzen erfolgt nicht.

Außerdem muß bei dem bekannten Verfahren ein Referenzsignal ohne Pumpimpuls ausgewertet werden, um die Phasenverschiebung messen zu können.

Aus der EP-A2-0072330 ist ein Verfahren bekannt, bei dem eine Messung des Druckes im Untersuchungsobjekt durchgeführt wird. Hierzu werden im Untersuchungsobjekt ausschließlich durch Beschallung mit Ultraschall Blasen erzeugt. Eine niederfrequente Ultraschallquelle im Bereich unter ca. 100 KHz bis typischerweise um 20 KHz erzeugt in dem zu untersuchenden Objekt in der Unterdruckphase Dampfblasen in gasfreien Flüssigkeiten oder Gasblasen, falls gelöste Gase vorhanden sind.

Die Ultraschalleistung wird hierbei so lange erhöht, bis im zu untersuchenden Körper Kavitationsblasen entstehen. Solche Blasen können sehr groß sein (mit bloßem Auge leicht sichtbar), im Schallfeld gefangen bleiben und ein Embolierisiko darstellen. Werden sie im Gewebe erzeugt, sind Begleitreaktionen wie bei der Taucherkrankheit zu erwarten. Durch die unvermeidlichen, vagabundierenden, tieffrequenten Ultraschallwellen sind insbesondere Lungenschäden zu befürchten.

Die EP-A2-0064399 beschreibt ein erfahren zur Bestimmung des Ultraschalldämpfungs-bzw. Absorptionskoeffizienten im Gewebe. Dazu wird die zeitliche oder in Ausbreitungsrichtung räumliche Änderung der Mittenfrequenz des Rückstreuspektrums berechnet. Diese Mittenfrequenz verschiebt sich mit wachsender Laufstrecke des Ultraschallimpulses wegen der etwa frequenzproportionalen Dämpfung langsam zu tieferen Frequenzen. Dabei ist die Verschiebung von $f_T$ nach $f_C$ und $f_R$ relativ gering.

Bei dem Verfahren gemäß US-A-3640271 werden Blutdruck und Strömungsgeschwindigkeit gemessen. Dazu werden Einzelblasen von kontrollierter Größe im Bereich von 10 bis 100 μm Durchmesser injiziert und ihre Resonanzfrequenz vor und nach der Injektion bestimmt. Das erfolgt entweder mit einem gedämpften Wandler und einem Frequenzsweep oder mit einer Stoßanregung aus einem schwach gedämpften Wandler. Der Blasengröße entsprechend müssen Frequenzen im Bereich von 60 bis 600 KHz, das heißt Wellenlängen von 2,5 bis 25 mm verwendet werden. Die benutzten Blasen sind groß, so daß sie die Kapillaren nicht passieren können. Die Geschwindigkeit der Blasen wird mittels Dopplereffekt oder aus der Laufzeit zwischen zwei Punkten gemessen.

B. Aus der Literaturstelle L. Germain, J.O.N. Cheeke, J. Acoust. Soc. Am. 83 (1988) 942 ist bekannt, die Bildqualität unter Ausnützung von harmonischen Vielfachen der Anregungsfrequenz in der Ultraschallmikroskopie zu verbessern. Zu diesem Zweck müssen jedoch Ultraschallwellen mit sehr großer Amplitude eingestrahlt werden, um auf dem Weg in das Untersuchungsgebiet nichtlineare Schwingungen zu erzeugen, wobei durch diese Nichtlinearität eine Übertragung von Energie aus den Schwingungen mit der Grundfrequenz in höher harmonische Schwingungen erfolgt.

Diese Literaturstelle ebenso wie die Literaturstelle Journal of the Acoustical Society of America, Band 69, Nr. 4, April 1981, S.1210 - 1212, W.K. Law et al. bezieht sich auf die nichtlineare Ausbreitung von Ultraschall, die in Wasser und Gewebe erst bei hohen Intensitäten auftritt.

Bei nichtlinearer Ausbreitung entstehen keine subharmonischen Schwingungen und harmonische Schwingungen entstehen erst nach einer Mindestlaufstrecke von einigen Zentimeter im Medium.

Diese Verfahren können jedoch bei der Ultraschalluntersuchung mit Frequenzen beispielsweise im Bereich von 1 bis 10 MHz von Objekten, die gegen hohe Schallintensitäten nicht widerstandsfähig sind, wie insbesondere biologische Gewebe, nicht angewendet werden.

Der Erfindung liegt die Aufgabe zugrunde, den Anwendungsbereich von Ultraschallverfahren für begrenzt gegen Schallintensität resistente Objekte, insbesondere biologische Gewebe, zur selektiven Bilddarstellung und Auswertung des Dopplerspektrums zu erweitern und Schaltungen zur Durchführung dieser Verfahren zu schaffen.

Die Aufgabe wird gelöst durch ein Verfahren gemäß den Ansprüchen 1 und 2 sowie durch eine Schaltung gemäß Anspruch 10. Vorteilhafte Ausgestattungen sind Gegenstand der Unteransprüche.

Durch das Einbringen von Materialien oder Medien in das zu beschallende Untersuchungsgebiet, die eine Nichtlinearität erzeugen, ist es überraschenderweise möglich, schon bei geringen Schallintensitäten, die nicht schädlich sind, zusätzlich zur Anregungsfrequenz fo intensive und stark frequenzverschobene Streu- und/oder Transmissionssignale zu erhalten. Diese Streu- und/oder Transmissionssignale sind insbesondere bei den Harmonischen ($2 f_o$, $3 f_o$...), Subharmonischen ($1/2 f_o$, $1/3 f_o$, $3/4 f_o$...) und Ultraharmonischen ($3/2 f_o$, $5/4 f_o$...) der Anregungsfrequenz intensiv. Mit diesem Verfahren kann man mit niedrigen Frequenzen einstrahlen, so daß man eine größere Eindringtiefe erlangt und Empfangssignale höherer Frequenzen auswerten.

In vorteilhafter Weise ist eine selektive Auswertung der von den eingegebenen Materialien oder Medien · beeinflußten Signalanteile sowie eine selektive Darstellung der von diesen Mitteln gefüllten Bereiche möglich, ohne daß eine bisher erforderliche Differenzbildung zwischen zwei oder mehr vor und nach der Applikation der Materialien oder Medien aufgenommenen Zustände vorgenommen wird. Insbesondere kann der hervorgerufene Dopplereffekt frei von Artefakten ausgewertet werden.

Mit Vorteil werden in das Untersuchungsgebiet nichtlineare Streukörper eingebracht. In das Untersuchungsgebiet können aber auch ein nichtlineares Ultraschallkontrastmittel in Form einer Lösung oder Suspension und insbesondere Mikrobläschen oder Mikrobläschen erzeugende Mittel eingebracht werden.

Das Einbringen einer Mikrobläschensuspension mit einer Konzentration von $10^{-3}$ Gew% bis 30 Gew% Trockensubstanz im Suspensionsmedium führt zu guten Ergebnissen, wobei überraschenderweise die niedrige untere Grenze von $10^{-3}$ Gew% mit dem erfindungsgemäßen Verfahren und der erfindungsgemäßen Schaltung erreicht wird.

Mit Vorteil wird bei dem erfindungsgemäßen Verfahren der Schallwandler mittels eines Funktionsgenerators angeregt, mit dem HF-Bursts mit einstellbarer Amplitude und einstellbarer Mittenfrequenz ($f_T$) im Bereich von 0,3 MHz bis 22 MHz vorzugsweise 1 MHz bis 11 MHz und mit 0,5 bis 20, vorzugsweise 1-5 Perioden erzeugt werden. Dabei hat sich als besonders vorteilhaft herausgestellt, Frequenzen auszuwerten, die kleiner sind, als die Schallwandler (Transmitter)-Mittenfrequenz $f_T$.

Bei der Auswertung ist es vorteilhaft, mittels einer rechnergesteuerten Torschaltung wenigstens einen Zeitabschnitt auszuwählen und das zugehörige Frequenzspektrum analog oder digital zu bestimmen. Dabei werden die Zeitfensterlänge und Anzahl der Perioden pro Burst zwischen optimaler Frequenzauflösung und optimaler Ortsauflösung eingestellt.

Mit dem erfindungsgemäßen Verfahren können mit Vorteil Dopplereffekte bei Harmonischen der Anregungsfrequenz und bei den Mischprodukten wie oberes Seitenband bei 2-Frequenz-Anregungen ausgewertet werden. Dies läßt die Darstellung langsamerer Flüsse ohne Störungen durch Gefäßwandbewegungen zu.

Darüber hinaus ergibt sich bei der Auswertung von harmonischen Signalanteilen oder von Signalen im oberen Seitenband eine verbesserte Eindringtiefe und/oder räumliche Auflösung, die bei der bildlichen Darstellung und bei Dopplermessungen sehr vorteilhaft ist.

Die erfindungsgemäße Schaltung zur Durchführung des im vorstehenden beschriebenen Verfahrens weist einen Funktionsgenerator auf, dessen Ausgang über einen vom Funktionsgenerator synchronisierten S/E (Sender-Empfänger) Schalter, dem ein Signalverarbeitungssystem nachgeschaltet, ist, mit dem Schwinger eines akustisch hochbedämpften, elektrisch angepaßten, breitbandigen Wandlerelementes verbunden ist.

Bei einer anderen Ausführungsform der Schaltung ist der Funktionsgenerator mit dem Eingang eines Wandlers verbunden, dessen Ausgang an ein Signalverarbeitungssystem angeschlossen ist.

Im zuerst genannten Fall wird bei Schaltung "senden" des S/E Schalters der vom Funktionsgenerator erzeugte Burst auf den Schwinger des Wandlers gegeben und das vom Wandler empfangene Signal wird bei eingeschalteter Stellung "empfangen" der S/E Schaltung an das Auswertesystem weitergegeben. Im zweiten Fall sind bei dem Wandler Eingang und Ausgang getrennt, so daß ein S/E Schalter nicht erforderlich ist.

EP 0 357 164 B1

Mit besonderem Vorteil wird ein Wandlerelement verwendet, dessen Mittenfrequenz $f_T$ größer ist als die obere Grenze des Arbeitsbereiches. Dieses Wandlerelement ist so ausgebildet, daß die von diesem abgestrahlte Schallintensität als Funktion der Frequenz im Frequenzbereich unterhalb der Anregungs- oder Mittenfrequenz $f_T$ eine positive erste Ableitung nach der Frequenz aufweist, die insbesondere im Arbeitsbereich annähernd konstant ist oder daß die Schallintensität die im Arbeitsbereich selbst einen konstanten Wert hat. Durch diesen nahezu gradlinigen Frequenzgang im Arbeitsbereich kann ein ähnlicher Frequenzgang, insbesondere der Dämpfung, im beschallten Untersuchungsgebiet weitgehend ausgeglichen werden. Durch diese Schaltung und den verwendeten Wandler ist ein Wechsel der zur Untersuchung verwendeten Frequenz ohne Schallkopfwechsel möglich. Außerdem kann bei der Auswertung von Spektren zur Materialcharakterisierung insbesondere bei der Gewebecharakterisierung das jeweils optimale Verhältnis von räumlicher Auflösung und Frequenzauflösung gewählt werden.

Das erfindungsgemäße Verfahren kann mit Vorteil mittels einer Schaltung durchgeführt werden, die einen Multielementenwandler mit phasenverzögert beaufschlagten Wandlerelementen aufweist, um ein phase-array oder ein dynamisch fokusiertes Verfahren durchzuführen. Bei dieser Schaltung ist der Ausgang eines Funktionsgenerators über einen n-Wege-Signalteiler, n rechnergesteuerte Verzögerungsschaltungen und n, vom Funktionsgenerator oder einem Rechner gesteuerte S/E Schalter mit den Eingängen von n akustisch hochbedämpften elektrisch angepaßten breitbandigen Wandlerelementen verbunden, deren Ausgänge über n S/E jeweils an einen m-Wege-Signalteiler angeschlossen sind. Diese m-Wege-Signalteiler sind jeweils über m Verzögerungsschaltungen und m feste oder variable Schaltungen zur Frequenzbandselektion und ferner über eine Schaltung zur phasenrichtigen Summation und ggf. Signalaufteilung an ein System zur selektiven Weiterverarbeitung von m-Frequenzbändern angeschlossen.

Bei einer weiteren Lösung der der Erfindung zugrunde liegenden Aufgabe wird in das zu beschallende Untersuchungsgebiet ein Material eingebracht, mit dem in diesem Gebiet nichtlineare Schwingungen durch eingestrahlte Ultraschallwellen erzeugt werden, ein breitbandig, akustisch hochbedämpfter, elektrisch angepaßter Ultraschallwandler mit einem oder mehreren, einzeln oder in Gruppen zusammengefaßt, ansteuerbaren Wandlerelementen wird mittels zweier HF-Bursts angeregt, deren jeweilige Anregungsfrequenz voneinander verschieden und kleiner als die Hälfte der Frequenzobergrenze des Arbeitsbereiches ist und aus dem vom Ultraschallwandler empfangenen, aus dem Untersuchungsgebiet reflektierten oder aus diesen zurückgestreuten Ultraschallsignalen werden Signalkombinationen der beiden Anregungsfrequenzen, insbesondere deren Summen- oder Differenzfrequenz ausgewertet.

Dabei wird durch Einstrahlung von zwei voneinander getrennten Signalen ein stärkeres Empfangssignal mit einer Frequenz der Kombination der Frequenzen der eingestrahlten Signale, insbesondere der Summen- oder Differenzfrequenz erhalten. Dabei ist insbesondere die Summenfrequenz wegen der erzielbaren höheren räumlichen Auflösung von Interesse. Bei diesem Verfahren kann ein Wandlerelement mittels zweier HF-Bursts angeregt werden. Es besteht aber auch die Möglichkeit, zwei getrennte Wandlerelemente jeweils mit einem HF-Burst anzuregen, wobei die Mittenfrequenzen dieser HF-Bursts verschieden und kleiner als die Hälfte der Obergrenze der Frequenz des Arbeitsbereichs sind.

Durch die erfindungsgemäß erzeugte Nichtlinearität wird beispielsweise mit zwei tieffrequenten Signalen, z.B. $f_o \approx f_p \approx 2$ MHz ein stärkeres Empfangssignal bei $f_o + f_p$, d.h. bei etwa 4 MHz erhalten, als wenn man mit der gleichen Gesamtleistung $I_o$, $I_p$ nur ein Sendesignal der Frequenzen $f_o + f_p$ verwendet. Dieses Phänomen ermöglicht eine höhere Eindringtiefe bei hohen Beobachtungsfrequenzen.

Als Materialien oder Medien, die die Nichtlinearität erzeugen, können die gleichen wie beim Verfahren zur Auswertung der harmonischen Frequenzen der Anregungsfrequenz verwendet werden. Es können im wesentlichen die gleichen Schaltungselemente mit Zusatz eines zweiten HF-Generators verwendet werden.

Bei der Schaltung mit einem Multi-Elementen-Wandler wird zur Reduzierung der in das Untersuchungsgebiet eingestrahlten mittleren Leistung das zweite Signal immer nur in die jeweilige Richtung des ersten Signals gesandt und beginnt etwa 1 bis 2 Perioden früher und dauert bis zum Ende des ersten Burst Signals. Um dieses zu erreichen, wird das zweite Signal vom zweiten Generator durch entsprechende Verzögerungsschaltungen so beeinflußt, daß es nach Durchlauf des S/E Schalters auf die gleichen Wandlerelemente im Schallkopf gelangt und in die gleiche Richtung wie das erste Sendesignal ausgestrahlt wird. Die Schaltungsmatrix empfängt dann Signale bei der Frequenzsumme. Dabei wird der S/E Schalter vom länger dauernden zweiten Sendersignal gesteuert.

Ausführungsbeispiele der Erfindung sollen in der folgenden Beschreibung unter Bezugnahme auf die Figuren der Zeichnungen erläutert werden.

Es zeigen :

Fig. 1      ein Blockschaltbild,
Fig. 2      eine schematische Schnittdarstellung eines Probengefäßes,

<div align="center">4</div>

Fig. 3      eine Darstellung der Schall-Leitungskurve des Wandlers als Funktion der Frequenz,

Fig. 4-9      graphische Darstellungen der Rückstreusignale und

Fig. 10      ein weiteres Blockschaltdiagramm.

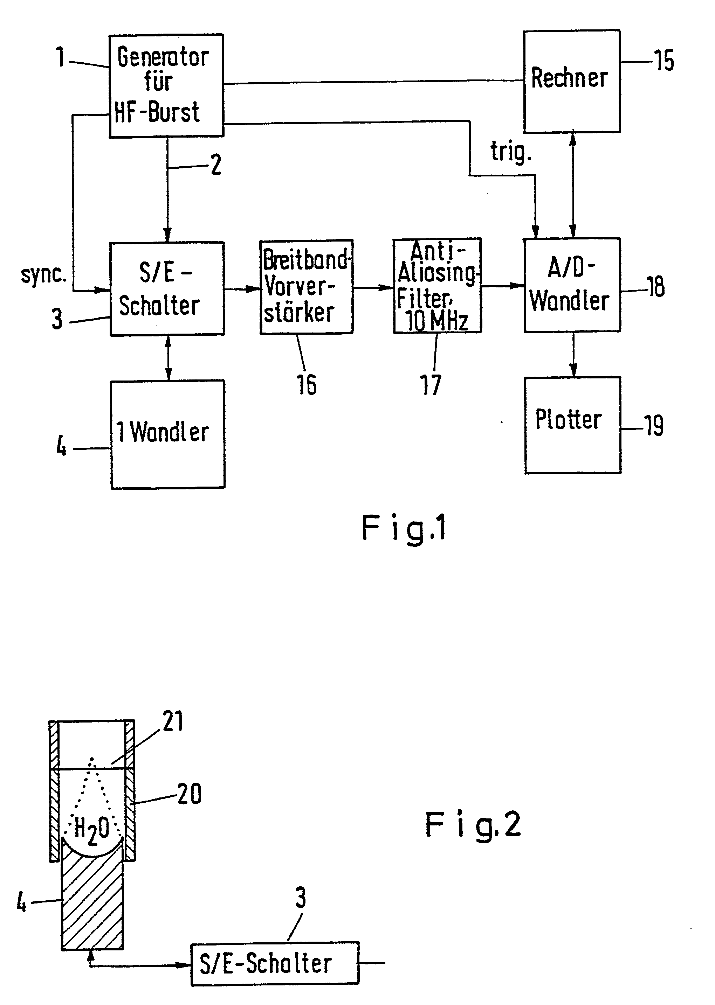

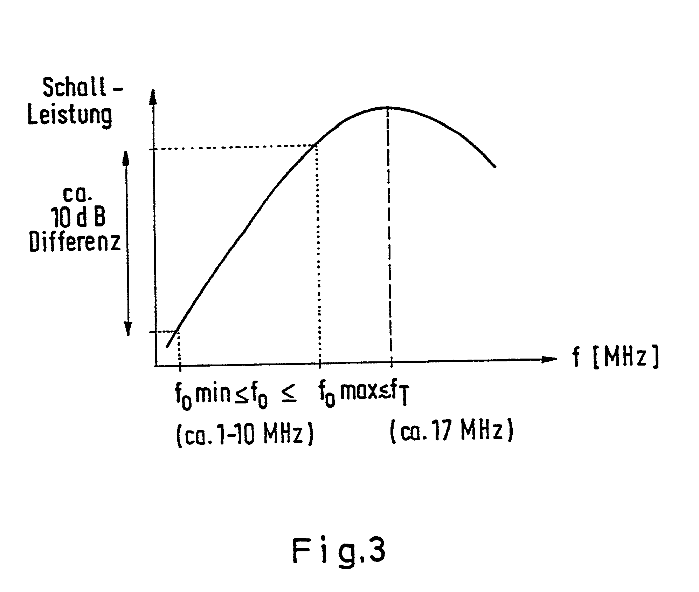

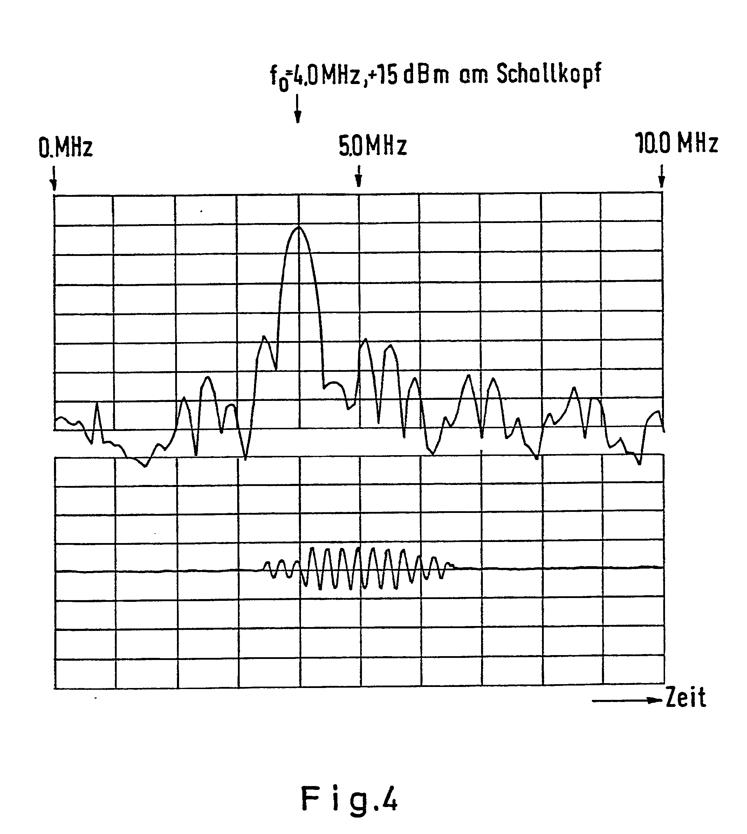

Zur Herstellung der in den Fig. 4 - 9 dargestellten, zur Weiterverarbeitung bereitgestellten Signale wird die in Fig. 1 dargestellte Schaltung zusammen mit dem in Fig. 2 dargestellten Probengefäß verwendet, wobei der breitbandige Schallkopf die in Fig. 3 dargestellte Leistungscharakteristik aufweist.

Periodisch wiederholte, elektrische Sendepulse — HF-Bursts — variabler Frequenz fo im Arbeitsbereich $f_{o\,min}...f_{o\,max}$ ($f_{o\,min}$ = 0,3 MHz < $f_o$ < $f_{o\,max}$ = 22 MHz) und variabler Bandbreite, gegeben durch die Anzahl n der Sinus-Perioden je Burst: 0,5 < n < 20 mit einstellbarer Amplitude werden von einem Funktionsgenerator 1 erzeugt, der vom zentralen Rechner 15 gesteuert wird. Dieser zentrale Rechner 15 steuert sowohl den Ablauf der Messung als auch deren Auswertung. Der Ausgang 2 des Generators 1 führt zu einem Sende-Empfangs-Schalter 3, der, wie schematisch dargestellt, vom Generator 1 synchronisiert wird. Der S/E Schalter 3 kann auch direkt vom Rechner 15 gesteuert werden. Der Ausgang 2 des S/E Schalters 3 ist mit einem breitbandigen, angepaßten und fokusierten Wandlerelement 4 verbunden. Die besonderen Merkmale dieses Wandlerelementes 4 sind schematisch in Fig. 3 dargestellt. Dieser Wandler weist eine große Breitbandigkeit ohne störende Resonanzen im Arbeitsbereich auf, ferner eine gute elektrische und akustische Impedanzanpassung und eine Transmitter-Mittenfrequenz $f_T > f_{o\,max}$. Beim beschriebenen Beispiel ist $f_t$ = 17 MHz. Dieser Wandler kann auch räumlich und elektrisch getrennte Sende- und Empfangswandlerelemente aufweisen. In diesem Fall wird der S/E Schalter 3 entbehrlich. Mit Vorteil kann zusätzlich ein weiteres Wandlerelement zur Aussendung eines zweiten unabhängigen Hochfrequenzsignals vorgesehen sein.

Das vom Wandlerelement 4 aufgenommene Signal wird über den umgeschalteten S/E Schalter einem Breitbandvorverstärker 16 zugeführt, dem, bei digitaler Frequenzanalyse, ein Anti-Aliasing-Filter 17 nachgeschaltet ist. Der Breitbandvorverstärker 16 weist eine Bandbreite > $f_o$ max auf. Der Filter 17 hat beispielsweise eine Abschneidefrequenz von 10 MHz. Dem Filter 17 ist ein schneller A/D-Wandler nachgeschaltet, in dem das Signal, beispielsweise mit einer Nyquistfrequenz von 12,5 MHz, digitalisiert wird. Die weitere Verarbeitung der Signale erfolgt in einem digitalen Speicheroszilloskop und im zentralen Rechner. Dem A/D Wandler 18 ist ein Plotter 19 nachgeschaltet.

Fig. 1 zeigt, daß der A/D-Wandler vom Funktionsgenerator 1 getriggert wird.

Das digitalisierte Signal wird gespeichert und in an sich bekannter Weise weiterverarbeitet. Es steht insbesondere für notwendige Korrekturen zur Verfügung. Vor der A/D-Wandlung kann auch ein Signal abgezweigt werden, das erst nach analoger Weiterverarbeitung digitalisiert wird.

Fig. 2 zeigt schematisch die Geometrie des Probengefäßes 20, mit dem die im folgenden dargestellten Messergebnisse erzielt wurden.

Wie Fig. 2 zeigt, ist im Probengefäß 20 der Schallkopf 4 angeordnet. Es handelt sich um einen 17 MHz-Schallkopf, der breitbandig, angepaßt und fokusiert ist. Im Probengefäß 20 befindet sich Wasser. Zwei Folien 21 trennen einen Probenbereich ab, in dem 10 mg Ultraschallkontrastmittel in 3 ml $H_2O$ gelöst sind.

Die im Messbereich zwischen den Folien 21 reflektierten und/oder zurückgestreuten Signale enthalten besondere Anteile, die durch eine Wechselwirkung zwischen dem Sendepuls (bei $f_o$) und dem in das Meßobjekt eingebrachten nichtlinearen Kontrastmittel erhalten wurden.

Fig. 3 zeigt schematisch das Frequenzband des Wandlerelementes im Schallkopf. Es ist zu erkennen, daß im Arbeitsbereich der Frequenzgang des Schwingers im Schallkopf quasi linear ist. Der Frequenzgang im Arbeitsbereich kann zum Ausgleich eines ähnlichen Frequenzganges im Prüfkörper benutzt werden. Der Frequenzgang im Prüfkörper kann aber auch nachträglich durch eine Gewichtung korrigiert werden.

Bei der Messung wird im Zeitbereich mittels einer nicht dargestellten, rechnergesteuerten Torschaltung ein interessierender Zeitabschnitt ausgewählt. Es können auch mehrere Zeitabschnitte ausgewählt werden. Mittels einer FFT-Schaltung (Fast Fourier Transformation) wird das zugehörige Spektrum berechnet und Beispiele derartiger Spektren sind in den Fig. 4-9 dargestellt. Durch die Wahl einer entsprechenden Zeitfensterlänge kann zwischen optimaler Frequenzauflösung und optimaler Ortsauflösung gewählt werden. In den Fig. 4-8 ist jeweils über dem Zeitfenster das Spektrum dargestellt. Um die Spektralkomponenten in diesen Fig. deutlich herauszuheben, wurde ein langes Zeitfenster, d.h. eine schlechte Ortsauflösung gewählt. Fig. 4 veranschaulicht den Zeitverlauf des Sendepulses nach einer Reflektion am Einkoppelfenster ohne Kontrastmittel. $f_o$ = 4,0 MHz, + 15 dBm am Schallkopf. Es ist ein deutliches Signal bei 4 MHz zu erkennen. Das im oberen Teil der Fig. 4 dargestellte Signal ist ein gemitteltes Leistungsspektrum, welches hinter dem Tiefpaßfilter mit einer Nyquistfrequenz von 50 MHz erhalten wurde.

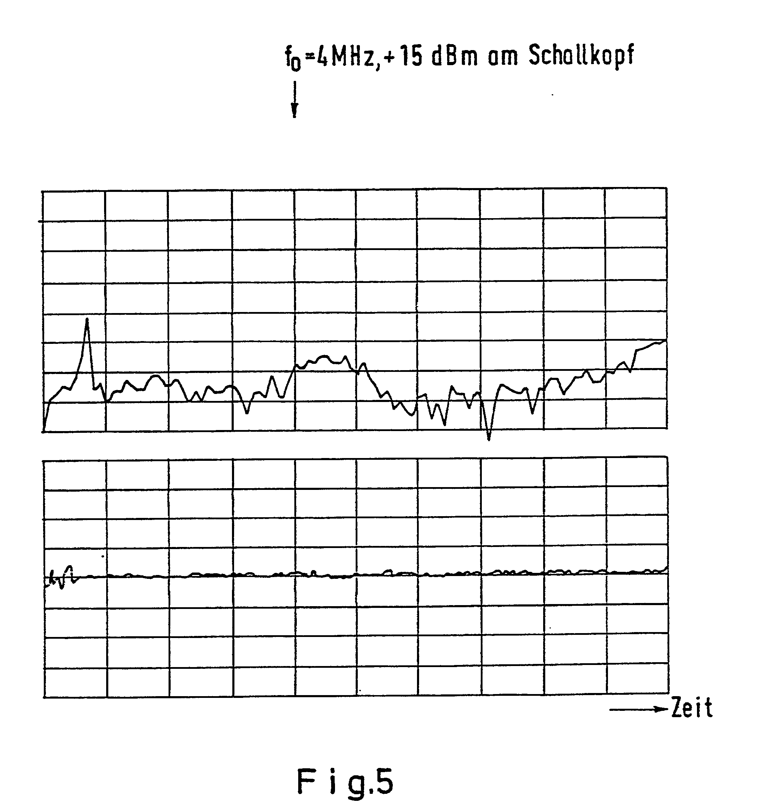

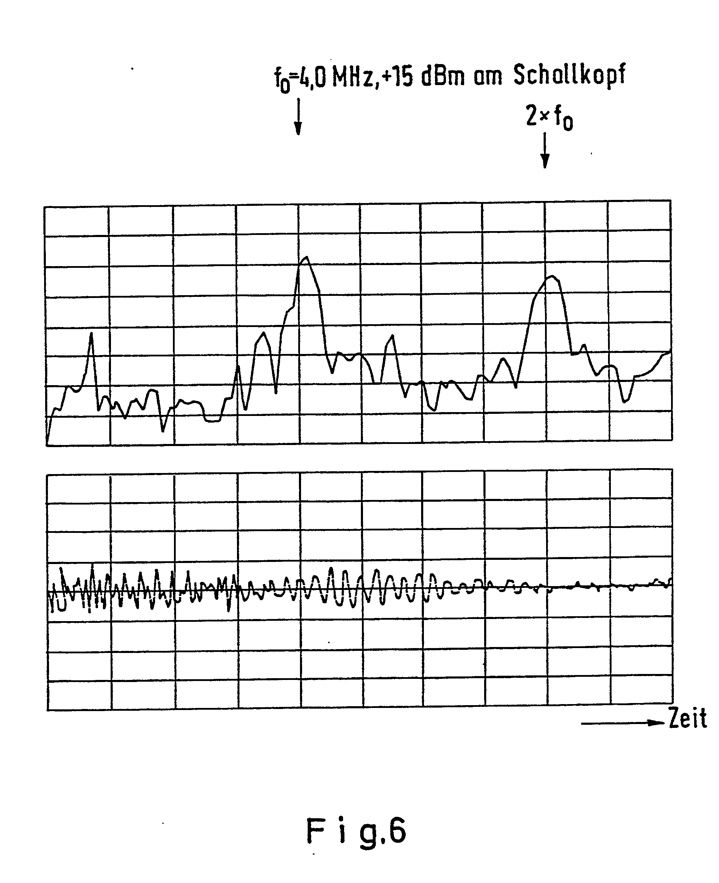

In Fig. 5 ist das Rückstreusignal aus der Probenkammer ohne Ultraschallkontrastmittel dargestellt. Fig. 6 zeigt das Rückstreusignal sieben Minuten nach Zugabe von 10 mg Kontrastmittel in 3 ml $H_2O$. Es ist ein deutlicher Peak bei 2 × $f_o$ zu erkennen.

EP 0 357 164 B1

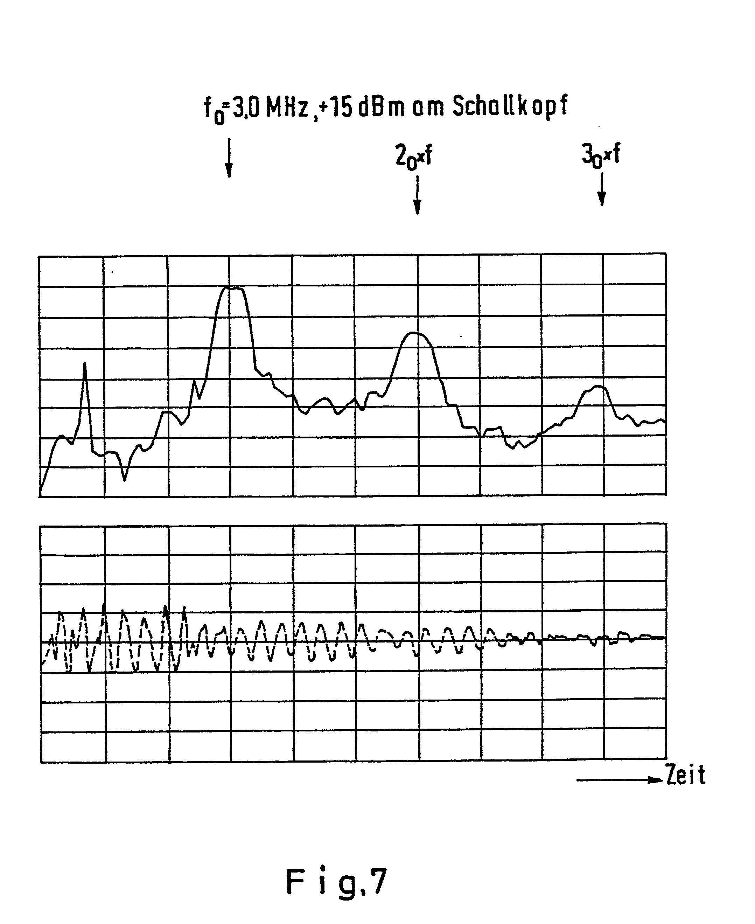

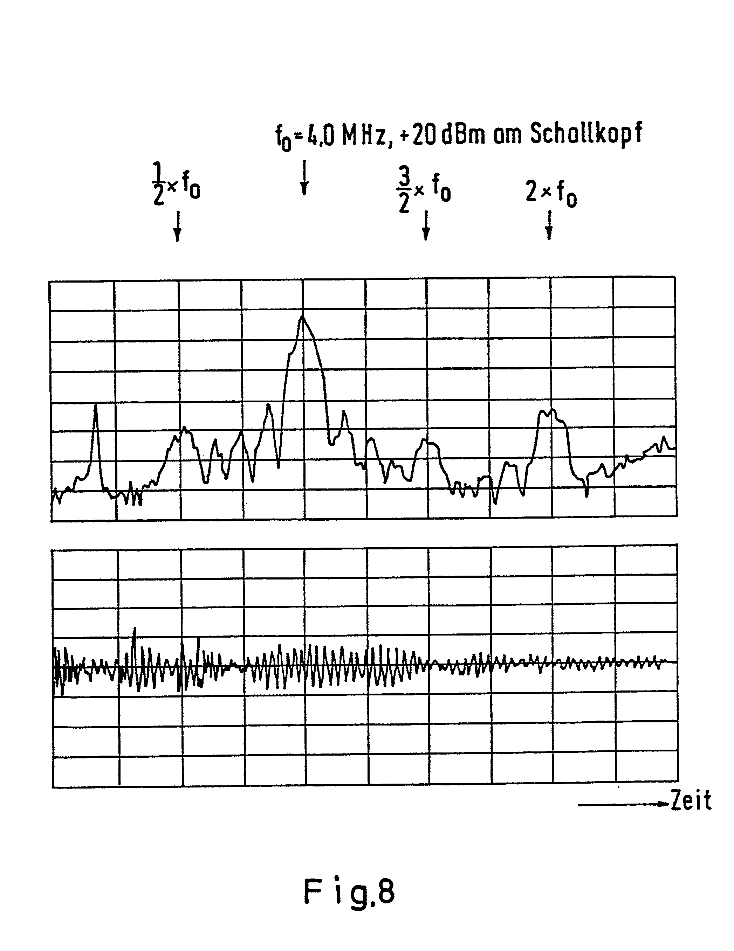

Fig. 7 zeigt eine Messung nach 21 Minuten unter den in Fig. 5 dargestellten Bedingungen. Es wurde mit einer Frequenz $f_o = 3$ MHz gearbeitet. Das empfangene Spektrum zeigt deutlich die erste und zweite Harmonische bei 6,0 und 9,0 MHz. Fig. 8 veranschaulicht das Rückstreusignal 15 Minuten nach Zugabe eines Ultraschallkontrastmittels kleinerer Konzentration. Es wurde mit einer Frequenz fo von 4 MHz + 20 dBm am Schallkopf gearbeitet. Das im oberen Teil der Fig. 8 dargestellte Spektrum zeigt mit höherer Frequenzauflösung Subharmonische bei 1/2 $f_o$, Ultraharmonische bei 3/2 $f_o$ und die erste Harmonische bei 2 $f_o$.

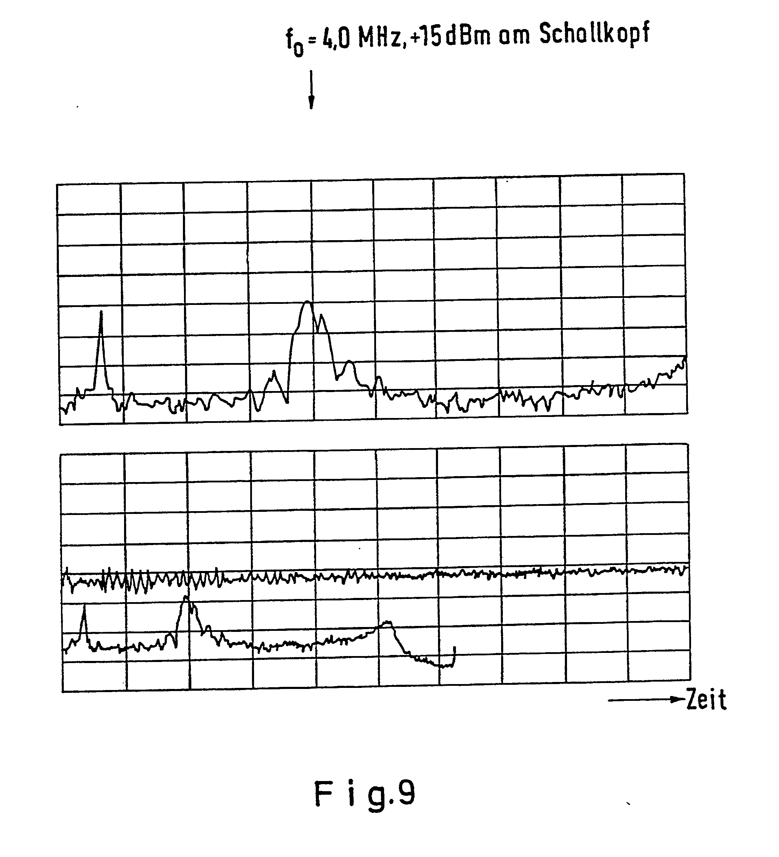

Fig. 9 zeigt ein Rückstrahlsignal von linearem Ultraschallkontrastmittel $f_o = 4$ MHz + 15 dBm am Schallkopf. Das Spektrum zeigt nur eine Rückstreuung bei der Anregungsfrequenz.

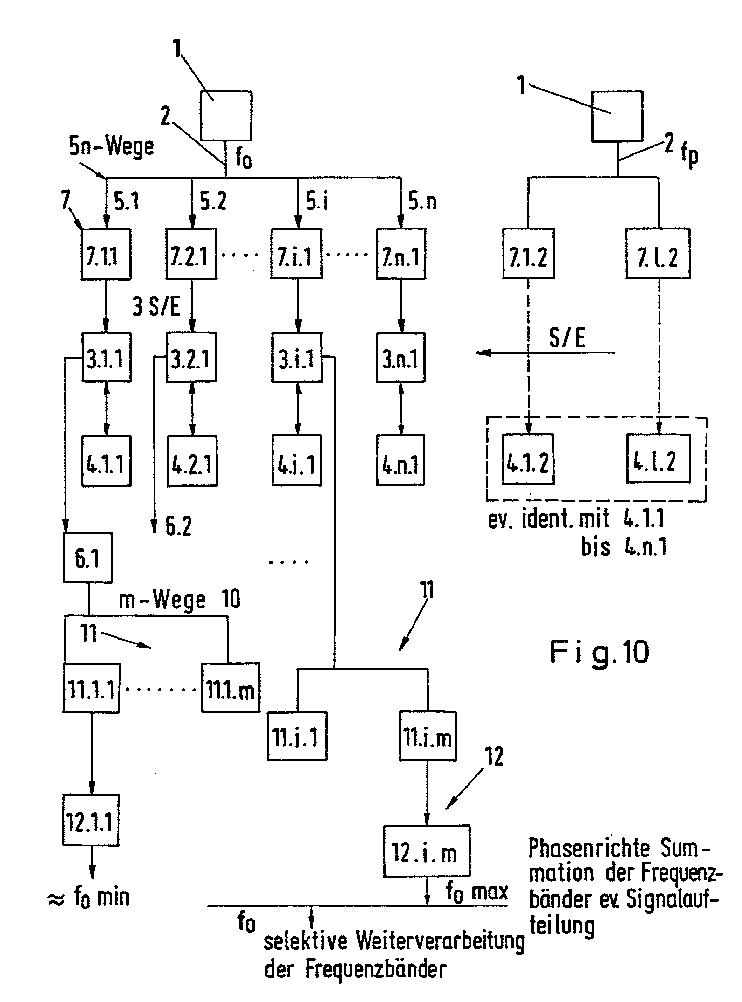

Es ist zu erkennen, daß die dargestellten Spektren deutliche Amplituden in Frequenzbereichen aufweisen, die im Sendespektrum nicht vorkommen, wenn eine Wechselwirkung mit einem nichtlinearen Kontrastmittel erfolgt ist. Es können Spektralveränderungen ausgewertet werden, die durch einen Dopplereffekt bedingt sind. Um die bei den geschilderten Ausführungsbeispielen verwendete Schaltung für bildgebende Ultraschallverfahren zu nutzen, sind zusätzliche Komponenten vorgesehen, falls ein Schallkopf vom Typ "Phased array" oder ein dynamischer fokussierter Schallkopf verwendet werden. Ein derartiges Schaltbild ist in Fig. 10 veranschaulicht.

Das Sendesignal des Funktionsgenerators 1 (Frequenz $f_o$) wird vom Ausgang 2 dem n-Wege-Signalteiler 5 zugeführt. Die Aufteilung erfolgt auf je einen Zweig pro Wandlerelement. Beim dargestellten Ausführungsbeispiel sind n-Wandler-Elemente 4 vorgesehen. Die Beaufschlagung dieser Wandlerelemente 4.1...4.n erfolgt über die Verzögerungsschaltungen 7.1...7.n und die vom Generator oder Rechner gesteuerten S/E Schalter 3.1...3.n. Die Verzögerungszeiten werden für jedes Wandlerelement vom Rechner so eingestellt, daß bei der gewählten Sendefrequenz die gewünschte Richtcharakteristik am Schallkopf entsteht. Die gleiche Richtcharakteristik wird vom Rechner durch entsprechende Verzögerungen im Empfängerteil eingestellt. Das von den Schallköpfen 4.1...4.n empfangene Signal wird über die S/E Schalter 3.1 Breitbandvorverstärker 6.1, 6.n zugeführt. Jeder Vorverstärker 6.1,... 6.n beaufschlagt einen m-Wege-Signalteiler 10, dem entsprechend gesteuerte oder eingestellte Verzögerungsschaltungen 11 jeweils nachgeschaltet sind, die Schaltungen 12 zur Frequenzbandselektion speisen. Nachgeschaltet sind Schaltungen zur phasenrichtigen Summation der Frequenzbänder und zur eventuellen Signalaufteilung. Daran schließt sich eine selektive Weiterverarbeitung der einzelnen Frequenzbänder mit den an sich bekannten Verfahren an.

Insbesondere erfolgt eine Auswertung der Frequenzen, die mit $f_o$ nicht identisch sind, beispielsweise 1/2 $f_o$, 2 $f_o$.

Die Verzögerungsschaltungen können variabel oder fest sein. Die Verteilung der empfangenen Signale auf m-Wege-Signalteiler erzeugt die gewünschte Zahl von Frequenzbändern, deren Lage und Breite mit Bandfiltern eingestellt werden. Alternativ kann die Aufteilung auch so erfolgen, daß das Empfangssignal mit einem vom Sendesignal abgeleiteten, je nach Frequenzband verschiedenen Hilfssignal so gemischt wird, daß die einzelnen Bänder in den Folgestufen mit einheitlichen Komponenten arbeiten können.

Das Frequenzband um $f_o$ liefert die üblichen Ergebnisse, während die anderen Bänder stark frequenzverschobene und nichtlineare Signalanteile aus Wechselwirkungen des Sendesignals mit den nichtlinearen Ultraschallkontrastmitteln enthalten.

Die weiteren Verarbeitungsschritte und Signalanalysen könnten in jedem beliebigen Frequenzkanal oder in mehreren parallelen Frequenzkanälen nach den bekannten Verfahren erfolgen.

Um zwei Sendefrequenzen $f_o$ und $f_p$ zu verwenden, ist der auf der rechten Seite der Fig. 10 dargestellte, zweite Generator vorgesehen, der über Signalteiler und Verzögerungsleitungen 15 an die S/E Schalter 3.1...3.n angeschlossen ist. Der zweite Generator 1 ermöglicht, mindestens den Raumbereich im Prüfobjekt zu beschallen, der durch die momentane Richtcharakteristik und das Empfangstor festgelegt wird. Der Aufbau kann derart sein, daß zusätzlich zu den beschriebenen breitbandigen Wandlerelementen sich im Schallkopf noch mindestens ein ebenfalls breitbandiger Sendewandler befindet, der vorzugsweise elektrisch von den übrigen getrennt ist und der von dem zweiten, unabhängigen Sendegenerator 1 gespeist wird. Die beiden Sendesignale können aber auch elektrisch so überlagert werden, daß die gleichen Wandlerelemente benutzt werden können.

**Patentansprüche**

1. Ultraschallverfahren zur Bilddarstellung und/gegebenenfalls zur Auswertung eines Dopplerspektrums von begrenzt gegen Schallintensität resistenten Objekten, bei dem

in das zu beschallende Untersuchungsgebiet des Objektes ein Mikrobläschen als Streukörper enthaltenes bzw. vor dem Beschallen Mikrobläschen erzeugendes Ultraschallkontrastmittel eingebracht wird, mit dem in diesem Gebiet nichtlineare Schwingungen dieser Mikrobläschen durch eingestrahlte Ultraschallwellen erzeugt werden,

6

ein breitbandiger, akustisch hoch bedämpfter, elektrisch angepaßter Ultraschallwandler mit einem Wandlerelement oder mit mehreren, einzeln oder in Gruppen zusammengefaßten, ansteuerbaren Wandlerelementen elektrisch mittels eines HF-Bursts der Anregungsfrequenz $f_o$ zum Beschallen des Untersuchungsgebietes angeregt wird, wobei $f_o$ im Bereich von 1 MHz bis 22 MHz liegt, und

das aus dem Untersuchungsgebiet reflektierte und aus diesem zurückgestreute Ultraschallsignal vom Ultraschallwandler empfangen wird und das empfangene Ultraschallsignal anschließend zur Auswertung weiterverarbeitet wird,

wobei aus dem reflektierten und zurückgestreuten Ultraschallsignal mindestens eine der Harmonischen, der Subharmonischen oder der Ultraharmonischen der Anregungsfrequenz $f_o$ und gegebenenfalls die Anregungsfrequenz $f_o$ ausgewertet wird.

2. Ultraschallverfahren zur Bilddarstellung und gegebenenfalls zur Auswertung eines Dopplerspektrums von begrenzt gegen Schallintensität resistenten Objekten, bei dem

in das zu beschallende Untersuchungsgebiet des Objektes ein Mikrobläschen als Streukörper enthaltenes bzw. vor dem Beschallen Mikrobläschen erzeugendes Ultraschallkontrastmittel eingebracht wird, mit dem in diesem Gebiet nichtlineare Schwingungen dieser Mikrobläschen durch eingestrahlte Ultraschallwellen erzeugt werden,

ein breitbandiger, akustisch hochbedämpfter, elektrisch angepaßter Ultraschallwandler mit einem Wandlerelement oder mit mehreren, einzeln oder in Gruppen zusammengefaßten, ansteuerbaren Wandlerelementen elektrisch mittels zweier HF-Bursts angeregt wird, deren jeweilige Anregungsfrequenz $f_o$ bzw. $f_p$ voneinander verschieden und kleiner als die Hälfte der Frequenzobergrenze des Arbeitsbereiches des Ultraschallwandlers ist, und

das aus dem Untersuchungsgebiet reflektierte und aus diesem zurückgestreute Ultraschallsignal vom Ultraschallwandler empfangen wird und das empfangene Ultraschallsignal anschließend zur Auswertung weiterverarbeitet wird,

wobei aus dem reflektierten und zurückgestreuten Ultraschallsignal die Summe oder die Differenz der beiden Anregungsfrequenzen ausgewertet wird.

3. Ultraschallverfahren nach Anspruch 1 oder 2 dadurch gekennzeichnet, daß das Ultraschallkontrastmittel eine Lösung, Emulsion oder Suspension ist.

4. Ultraschallverfahren nach Anspruch 3 dadurch gekennzeichnet, daß als Kontrastmittel eine Mikrobläschen-Suspension mit einer Konzentration von $10^{-3}$ Gew.-% bis 30 Gew.-% Trockensubstanz im Suspensionsmedium eingebracht wird.

5. Ultraschallverfahren nach einem der Ansprüche 1, 3 oder 4, dadurch gekennzeichnet, daß der Ultraschallwandler mittels mindestens eines Funktionsgenerators angeregt wird, mit dem HF-Bursts einstellbarer Amplitude und einstellbarer Anregungsfrequenz $f_o$ im Bereich von 1 MHz bis 22 MHz, vorzugsweise 1 MHz bis 11 MHz und mit 0,5 bis 20 Perioden, vorzugsweise 1 bis 5 Perioden erzeugt werden.

6. Ultraschallverfahren nach einem der Ansprüche 2 bis 4, dadurch gekennzeichnet, daß die zwei HF-Bursts von zwei Funktionsgeneratoren erzeugt und entweder einem Ultraschallwandlerelement oder zwei Ultraschallwandlerelementen zugeführt werden, und daß mit jedem Funktionsgenerator HF-Bursts einstellbarer Amplituden und einstellbarer Anregungsfrequenz $f_o$ bzw. $f_p$ im Bereich von 0,5 bis 20 MHz, vorzugsweise 1 bis 5 MHz mit 1 bis 25 Perioden, insbesondere 1 bis 10 Perioden erzeugt werden.

7. Ultraschallverfahren nach einem der Ansprüche 1 bis 5, dadurch gekennzeichnet, daß aus dem reflektierten und zurückgestreuten Ultraschallsignal Frequenzen ausgewertet werden, die kleiner sind als die Ultraschallwandler-Mittenfrequenz ($f_T$).

8. Ultraschallverfahren nach einem der Ansprüche 1 bis 5 und 7, dadurch gekennzeichnet, daß die Auswertung mittels einer rechnergesteuerten Torschaltung durchgeführt wird, wobei wenigstens ein Zeitfenster ausgewählt und das dazugehörige Frequenzspektrum analog oder digital bestimmt wird.

9. Ultraschallverfahren nach Anspruch 8, dadurch gekennzeichnet, daß die Länge des Zeitfensters und die Anzahl der Perioden pro Burst je nach gewünschter Frequenzauflösung und Ortsauflösung eingestellt wird.

10. Schaltung zur Durchführung des Ultraschallverfahrens nach mindestens einem der Ansprüche 1, 3, 4, 5, 7, 8 oder 9, dadurch gekennzeichnet, daß der Ultraschallwandler eine Anzahl n phasenverzögert beaufschlagter Ultraschallwandlerelemente aufweist, wobei

— der Ausgang (2) des Funktionsgenerators über einen n — Wege- Signalteiler (5),

— n rechnergesteuerte Verzögerungsschaltungen (7.1.1...7.n.1.)

— und n vom Funktionsgenerator (1) oder einem rechnergesteuerten Sende-/Empfangsschalter (3.1.1...3.n.1) mit

— den Eingängen der Ultraschallwandlerelemente verbunden ist, deren

— Ausgänge über die n Sende-/Empfangsschalter (3.1.1...3.n.1) an jeweils einem weiteren m-Wege-Signalteiler (10) angeschlossen sind, der die Signale auf eine Anzahl m Wege aufteilt, wobei die m-Wege-Signalteiler (10) jeweils über m Verzögerungsschaltungen (11), über
— m feste oder variable Schaltungen (12) zur Frequenzbandselektion und über
— eine Schaltung zur phasenrichtigen Summation und Signalaufteilung
— an einem System zur selektiven Weiterverarbeitung — einzeln oder parallel — von m Frequenzbändern
angeschlossen sind.

11. Schaltung nach Anspruch 10 zur Durchführung des Verfahrens nach Anspruch 2 bis 4, gekennzeichnet durch einen zweiten Funktionsgenerator (1), der über einen n-Wege-Signalteiler auf die n Verzögerungsschaltungen (7.1.1. bis 7.n.1.) aufschaltbar ist.

## Claims

1. Ultrasonic process for imaging and, optionally, for evaluation of a Doppler spectrum of objects having limited resistance to sound intensity, in which

there is introduced into the inspection zone, which is to be exposed to ultrasonic waves, of the object an ultrasonic contrast agent that contains microbubbles as scattering bodies or that produces microbubbles before the exposure, by means of which contrast agent non-linear vibrations of those microbubbles are produced in that zone by ultrasonic waves which are radiated in,

a wide-band, acoustically highly damped, electrically matched ultrasonic transducer having a transducer element or having several transducer elements, controllable individually or in groups, is excited electrically by means of an HF burst of excitation frequency $f_o$ in order to expose the inspection zone to ultrasonic waves, $f_o$ being in the range of from 1 MHz to 22 MHz, and

the ultrasonic signal reflected from the inspection zone and scattered back from that zone is received by the ultrasonic transducer, and the received ultrasonic signal is then processed further for evaluation,

there being evaluated from the reflected and back-scattered ultrasonic signal at least one of the harmonics, the subharmonics and the ultraharmonics of the excitation frequency $f_o$ and, optionally, the excitation frequency $f_o$.

2. Ultrasonic process for imaging and, optionally, for evaluation of a Doppler spectrum of objects having limited resistance to sound intensity, in which

there is introduced into the inspection zone, which is to be exposed to ultrasonic waves, of the object an ultrasonic contrast agent that contains microbubbles as scattering bodies or that produces microbubbles before the exposure, by means of which contrast agent non-linear vibrations of those microbubbles are produced in that zone by ultrasonic waves which are radiated in,

a wide-band, acoustically highly damped, electrically matched ultrasonic transducer having a transducer element or having several transducer elements, controllable individually or in groups, is excited electrically by means of two HF bursts, the excitation frequencies $f_o$ and $f_p$ of which are different and are less than half the upper frequency limit of the working range of the ultrasonic transducer, and

the ultrasonic signal reflected from the inspection zone and scattered back from that zone is received by the ultrasonic transducer, and the received ultrasonic signal is then processed further for evaluation,

there being evaluated from the reflected and back-scattered ultrasonic signal the sum or the difference of the two excitation frequencies.

3. Ultrasonic process according to claim 1 or claim 2, characterised in that the ultrasonic contrast agent is a solution, an emulsion or a suspension.

4. Ultrasonic process according to claim 3, characterised in that there is introduced as contrast agent a microbubble suspension having a concentration of from $10^{-3}$% by weight to 30% by weight dry substance in the suspension medium.

5. Ultrasonic process according to any one of claims 1, 3 or 4, characterised in that the ultrasonic transducer is excited by at least one function generator by means of which HF bursts of adjustable amplitude and adjustable excitation frequency $f_o$ in the range of from 1 MHz to 22 MHz, preferably from 1 MHz to 11 MHz, and with from 0.5 to 20 cycles, preferably from 1 to 5 cycles, are generated.

6. Ultrasonic process according to any one of claims 2 to 4, characterised in that the two HF bursts are generated by two function generators and fed either to one ultrasonic transducer element or to two ultrasonic transducer elements, and in that each function generator generates HF bursts of adjustable amplitude and adjustable excitation frequency $f_o$ or $f_p$ in the range of from 0.5 to 20 MHz, preferably from 1 to 5 MHz, with from 1 to 25 cycles, preferably from 1 to 10 cycles.

7. Ultrasonic process according to any one of claims 1 to 5, characterised in that, from the reflected and backscattered ultrasonic signal, frequencies that are lower than the average frequency ($f_T$) of the ultrasonic transducer are evaluated.

8. Ultrasonic process according to any one of claims 1 to 5 and 7, characterised in that the evaluation is carried out by means of a computer-controlled gate circuit, at least one time window being selected and the associated frequency spectrum being determined in analog or digital manner.

9. Ultrasonic process according to claim 8, characterised in that the length of the time window and the number of cycles per burst are adjusted according to the desired frequency resolution and spatial resolution.

10. Circuit for carrying out the ultrasonic process according to at least one of claims 1, 3, 4, 5, 7, 8 or 9, characterised in that the ultrasonic transducer has a number n of ultrasonic transducer elements which receive signals in phase-delayed manner,

— the output (2) of the function generator being connected by way of an n-way signal divider (5),

— n computer-controlled time-delay circuits (7.1.1...7.n.1)

— and n transmitter/receiver switches (3.1.1...3.n.1) controlled by the function generator (1) or by a computer to

— the inputs of the ultrasonic transducer elements,

— the outputs of which are connected, by way of the n transmitter/receiver switches (3.1.1...3.1.n), each to a further m-way signal divider (10) which divides the signals a number m of ways, the m-way signal dividers (10) being connected by way of m time-delay circuits (11), by way of

— m fixed or variable circuits (12) for frequency band selection and by way of

— a circuit for phase-correct summation and signal division

— to a system for the selective further processing — individually or in parallel — of m frequency bands.

11. Circuit according to claim 10 for carrying out the process according to any one of claims 2 to 4, characterised by a second function generator (1) which can be connected <u>via</u> an n-way signal divider to the n time-delay circuits (7.1.1...7.n.1).

## Revendications

1. Procédé à ultrasons pour la présentation d'images et/le cas échéant l'évaluation d'un spectre Doppler d'objets résistant de manière limitée à l'intensité acoustique, dans lequel procédé

un moyen de contraste pour ultrasons contenant des microbulles comme corps diffusants, produisant des microbulles avant l'exposition à l'action des ultrasons, est introduit dans le domaine de recherche de l'objet à l'objet à exposer à l'action des ultrasons, moyen de contraste avec lequel des oscillations non linéaires de ces microbulles sont engendrées dans le domaine précité par des ondes ultrasonores qui irradient celles-ci,

un convertisseur ultrason à large bande, fortement amorti acoustiquement et adapté électriquement, qui comprend un élément convertisseur ou plusieurs éléments convertisseurs excitables et agencés individuellement ou par groupes, est excité électriquement au moyen d'une impulsion HF à la fréquence d'excitation $f_0$ pour l'exposition à l'action des ultrasons du domaine de recherche, $f_0$ étant compris dans la gamme allant de 1 MHz à 22 MHz, et

le signal ultrason réfléchi à partir du domaine de recherche et diffusé en retour à partir de celui-ci est reçu par le convertisseur ultrason et le signal ultrasonore reçu est ensuite traité pour évaluation,

de manière qu'au moins l'un des harmoniques, des sous- harmoniques ou des ultra-harmoniques de la fréquence d'excitation $f_0$ et le cas échéant la fréquence d'excitation $f_0$ soit évalué à partir du signal ultrasonore réfléchi et diffusé en retour.

2. Procédé à ultrasons pour la présentation d'images et/le cas échéant l'évaluation d'un spectre Doppler d'objets résistant de manière limitée à l'intensité acoustique, dans lequel procédé

un moyen de contraste pour ultrasons contenant de microbulles comme corps diffusants ou produisant des microbulles avant l'exposition à l'action des ultrasons est introduit dans le domaine de recherche de l'objet à exposer à l'action des ultrasons, moyen de contraste avec lequel des oscillations non linéaires de ces microbulles sont engendrées dans le domaine précité par des ondes ultrasonores qui irradient celles-ci,

un convertisseur ultrason à large bande, fortement amorti acoustiquement et adapté électriquement, qui comprend un élément convertisseur ou plusieurs éléments convertisseurs excitables et agencés individuellement ou par groupes, est excité électriquement au moyen de deux impulsions haute fréquence, dont les fréquences d'excitation respectivement $f_0$ et $f_p$ diffèrent l'une de l'autre et sont inférieures à la moitié de la limite supérieure de la fréquence du domaine de fonctionnement du convertisseur ultrason, et

le signal ultrasonore réfléchi à partir du domaine de recherche et diffusé en retour à partir de celui-ci est reçu par le convertisseur ultrason et le signal ultrasonore reçu est ensuite traité pour évaluation,

de manière que la somme oy la différence des deux fréquences d'excitation soit évaluée à partir du signal ultrasonore réfléchi et diffusé en retour.

3. Procédé à ultrasons suivant la revendication 1 ou 2, caractérisé en ce que le moyen de contraste ultrason est une solution, une émulsion ou une suspension.

4. Procédé à ultrasons suivant la revendication 3, caractérisé en ce qu'il est introduit dans l'agent de suspension comme moyen de contraste une suspension à microbulles présentant une concentration allant de $10^{-3}$% en poids à 30% en poids de matières sèches.

5. Procédé à ultrasons suivant l'une quelconque des revendications 1, 3 ou 4, caractérisé en ce que le convertisseur ultrason est excité au moyen d'au moins un générateur de fonctions avec lequel sont engendrées des impulsions haute fréquence d'amplitude réglable et de fréquence d'excitation $f_o$ réglable dans la gamme allant de 1 MHz jusqu'à 22 MHz, de préférence de 1 MHz jusqu'à 11 MHz et avec 0,5 jusqu'à 20 périodes, de préférence de 1 à 5 périodes.

6. Procédé à ultrasons suivant une des revendications 2 à 4, caractérisé en ce que les deux impulsions haute fréquence de deux générateurs de fonctions sont engendrées et amenées soit à un élément de convertisseur ultrason, soit à deux éléments de convertisseur ultrason et en ce que, avec chaque générateur de fonctions sont engendrées des impulsions haute fréquence d'amplitudes réglables et ayant des fréquences d'excitation respectivement $f_o$ ou $f_p$ réglables dans la gamme allant de 0,5 à 20 MHz, de préférence de 1 à 5 MHz avec des périodes allant de 1 à 25, en particulier des périodes allant de 1 à 10.

7. Procédé à ultrasons suivant l'une des revendications 1 à 5, caractérisé en ce que les fréquences, qui sont inférieures à la fréquence moyenne ($f_T$) du convertisseur à ultrasons, sont évaluées à partir du signal ultrasonore réfléchi et diffusé en retour.

8. Procédé à ultrasons suivant l'une des revendications 1 à 5 et 7, caractérisé en ce que l'évaluation est effectuée au moyen d'un circuit-ET commandé par ordinateur, de manière qu'une fenêtre de temps au moins soit sélectionnée et que le spectre de fréquences correspondant soit déterminé de manière analogique ou numérique.

9. Procédé à ultrasons suivant la revendication 8, caractérisé en ce que la longueur de la fenêtre de temps et le nombre de périodes par impulsion sont réglés suivant les définitions de fréquence et de lieu désirées.

10. Circuit pour la mise en oeuvre du procédé à ultrasons suivant au moins l'une des revendications 1, 3, 4, 5, 7, 8 ou 9, caractérisé en ce que le convertisseur ultrason présente un nombre $\underline{n}$ d'éléments de convertisseur ultrason sollicités en retard de phase, la sortie (2) du générateur de fonctions étant connectée, par l'intermédiaire

— d'un diviseur de signal à $\underline{n}$ voies (5),

— de n circuits de retardement commandés par ordinateur (7.1.1...7.n.1.), et

— de $\underline{n}$ ou d'un circuit d'émission/de réception commandés respectivement par le générateur de fonctions (1) ou par ordinateur (3.1.1...3.n.1),

aux entrées des éléments des convertisseurs ultrason, dont des sorties sont raccordées, par l'intermédiaire des $\underline{n}$ circuits d'émission/de réception (3.1.1...3.n.1) à chaque fois un autre diviseur de signal à $\underline{m}$ voies (10), qui divise les signaux en un nombre $\underline{m}$ de voies, les diviseurs de signal à $\underline{m}$ voies (10) étant raccordés chacun par l'intermédiaire de $\underline{m}$ circuits de retardement (11), par l'intermédiaire de

— $\underline{m}$ circuits fixes ou variables (12) destinés à la sélection de bandes de fréquences et par l'intermédiaire

— d'un circuit destiné à la division de signaux et l'addition en phase à un système destiné au traitement ultérieur sélectif

— individuellement ou en parallèle — de $\underline{m}$ bandes de fréquences.

11. Circuit suivant la revendication 10 pour la mise en oeuvre du procédé suivant les revendications 2 à 4, caractérisé par un deuxième générateur de fonctions (1), qui peut être monté sur les $\underline{n}$ circuits de retardement (7.1.1. à 7.n.1.) par l'intermédiaire d'un diviseur de signal à $\underline{n}$ voies.

1 — Generator für HF-Burst

15 — Rechner

2

sync.

3 — S/E-Schalter

4 — 1 Wandler

16 — Breitband-Vorverstärker

17 — Anti-Aliasing-Filter, 10 MHz

trig.

18 — A/D-Wandler

19 — Plotter

F i g.1

21

20

$H_2O$

4

3 — S/E-Schalter

F i g.2

Fig.3

$f_0 = 4.0\,\text{MHz}, +15\,\text{dBm}$ am Schallkopf

0.MHz          5.0 MHz          10.0 MHz

Fig.4

$f_0 = 4\,MHz, +15\,dBm$ am Schallkopf

→ Zeit

# Fig.5

$f_0$=4,0 MHz,+15 dBm am Schallkopf

$2 \times f_0$

Zeit

# Fig.6

$f_0 = 3{,}0\ \text{MHz}, +15\ \text{dBm am Schallkopf}$

$2_0 \times f$    $3_0 \times f$

Zeit

F i g. 7

$f_0 = 4.0 \text{ MHz}, +20 \text{ dBm am Schallkopf}$

$\frac{1}{2} \times f_0$

$\frac{3}{2} \times f_0$

$2 \times f_0$

Zeit

# Fig.8

$f_0 = 4{,}0$ MHz, +15 dBm am Schallkopf

——→ Zeit

F i g.9

F i g. 10

Phasenrichte Summation der Frequenzbänder ev. Signalaufteilung